# EUROPÄISCHE PATENTSCHRIFT

(11) **EP 1 209 146 B1**
(45) Veröffentlichungstag und Bekanntmachung des Hinweises auf die Patenterteilung: **30.06.2004**
(21) Anmeldenummer: 01126430.6
(22) Anmeldetag: 08.11.2001
(51) Int. Cl.: C07C 209/48

(54) **Verfahren zur Hydrierung von Nitrilen zu primären Aminen an Raney-Katalysatoren**
Process for the hydrogenation of nitriles to primary amines using Raney-catalysts
Procédé d'hydrogenation de nitriles en amines primaires utilisant des catalyseurs de Raney

(30) Priorität: 16.11.2000 DE 10056840
(43) Veröffentlichungstag der Anmeldung: 29.05.2002
(73) Patentinhaber: BASF AKTIENGESELLSCHAFT, 67056 Ludwigshafen (DE)
(72) Erfinder: Ansmann, Andreas, 69168 Wiesloch (DE); Benisch, Christoph, 68165 mannheim (DE); Funke, Frank, 68161 Mannheim (DE); Ohlbach, Frank, 40219 Düsseldorf (DE); Merger, Martin, 67227 Frankenthal (DE)
(74) Vertreter: Isenbruck, Günter, Dr.

(56) Entgegenhaltungen:
- EP-A- 0 212 986
- WO-A-00/27525
- FR-A- 2 248 265
- GB-A- 653 348
- US-A- 4 375 003
- DATABASE WPI Section Ch, Week 198834 Derwent Publications Ltd., London, GB; Class E16, AN 1988-238148 XP002188943 & JP 63 170342 A (KAWAKEN FINE CHEM CO LTD), 14. Juli 1988 (1988-07-14)

## Beschreibung

Die Erfindung betrifft ein Verfahren zur Hydrierung von Nitrilen zu primären Aminen an einem aktivierten Raney-Katalysator.

Es ist bekannt, Nitrile und Iminonitrile in flüssiger Phase an Raney-Katalysatoren zu hydrieren.

EP-A-0 382 508 beschreibt die halbkontinuierliche Hydrierung von Polynitrilen in flüssiger Phase an Raney-Cobalt-Katalysatoren in Gegenwart von wasserfreiem Ammoniak.

US 5,869,653 beschreibt ein kontinuierliches Verfahren zur Hydrierung von Nitrilen an Raney-Cobalt-Katalysatoren in Abwesenheit von Ammoniak, wobei in Gegenwart katalytischer Mengen von Lithiumhydroxid und Wasser gearbeitet wird.

US 4,895,994 offenbart einen Raney-Katalysator mit einer BET-Oberfläche von 20 bis 80 m²/g und einem Makroporen-Anteil von 0,01 bis 70 Vol.-%, bezogen auf das Gesamtporenvolumen, der hergestellt wird durch Vermischen der Raney-Legierung mit einem hochmolekularen Polymer, Verformen der Mischung zu einem Formkörper, Calcinieren der Zusammensetzung zuerst bei 300 bis 700°C und anschließend bei 850 bis 1200°C in Gegenwart von Sauerstoff und Auslaugen von Aluminium aus dem calcinierten Formkörper durch Behandlung mit 6 N-NaOH bei 90 bis 100°C. Der so aktivierte Katalysator wird anschließend wiederholt mit Wasser gewaschen, bis der pH-Wert des Waschwassers < 9 beträgt. Der Raney-Katalysator wird unter anderem zur Hydrierung von Nitrilen zu Aminen eingesetzt.

EP-A-0 842 699 offenbart ein Verfahren zur Herstellung eines aktivierten, metallpulverfreien, Makroporen aufweisenden Metall-Festbettkatalysators nach Raney auf der Basis einer Legierung aus Aluminium und mindestens einem Metall der VIII. Nebengruppe des Periodensystems, mit den Schritten (1) Herstellen einer Knetmasse enthaltend die Legierung, ein Verformungsmittel, Wasser und einen Porenbildner, (2) Verformen der Knetmasse zu einem Formkörper, (3) Calcinieren des Formkörpers und (4) Behandeln des calcinierten Formkörpers mit einem Alkalimetallhydroxid. Nach der Behandlung des Formkörpers mit Alkalimetallhydroxid wird der aktivierte Katalysator solange mit Wasser gewaschen, bis der pH-Wert des Waschwassers auf 7,5 gesunken ist. Der so erhaltene Katalysator weist einen Gehalt an Makroporen von mehr als 80 Vol.-% auf. Der Katalysator wird zur Hydrierung von Nitrilen zu primären Aminen eingesetzt.

Ein derartiges Verfahren offenbart auch DE-A 44 46 907, wobei Polyvinylalkohol und Wasser oder Stearinsäure als Hilfsmittel eingesetzt werden.

Nachteilig an den halbkontinuierlichen oder kontinuierlichen Verfahren des Standes der Technik ist, daß entweder in Gegenwart von Ammoniak als Lösungsmittel oder in Gegenwart von basischen Alkalimetallverbindungen wie LiOH gearbeitet wird. Die Hydrierung in Gegenwart von Ammoniak bringt zusätzlichen technischen Aufwand, der mit der Abtrennung, Aufarbeitung und Rückführung des Ammoniaks verbunden ist, mit sich. Basische Alkalimetallverbindungen wirken korrosiv, ihr Einsatz erfordert daher ebenfalls zusätzlichen technischen Aufwand.

US 4,375,003 betrifft ein Verfahren zur Herstellung von primären Aminen aus aliphatischen Nitrilen und Wasserstoff in Gegenwart von Raney-Cobalt-Katalysatoren. Bei der Herstellung des Katalysators wird von einer binären Al/Co-Legierung mit einem Al-Gehalt im Bereich von 30 bis 70 Gew.-% ausgegangen. In den Beispielen werden verschiedene Nitrile, darunter 3-(N,N-Dimethylamino)propionitril in Abwesenheit von Ammoniak oder einer basischen Alkali- oder Erdalkaliverbindung hydriert. Der Katalysator wird mit Alkalimetallhydroxid-Lösung aktiviert und anschließend mit destilliertem Wasser gewaschen.

EP-A 0 212 986 offenbart ein Verfahren zur Herstellung von aliphatischen Polyaminen durch Hydrierung von aliphatischen Polynitrilen in Gegenwart von mindestens 5 Gew.-% eines flüssigen primären oder sekundären Amins an Raney-Cobalt bei 3,45 bis 69 bar Wasserstoffdruck und 50 bis 150 °C. Die Mitverwendung von Ammoniak oder einer basischen Alkali- oder Erdalkaliverbindung wird nicht erwähnt. In den Beispielen werden Nitrilotriacetonitril, Iminodiacetonitril und Ethylendiamintetraacetonitril an Raney-Cobalt hydriert. Der eingesetzte Raney-Cobalt-Katalysator wird aus einer Legierung enthaltend 60 Gew.-% Aluminium, 38 Gew.-% Cobalt und 2 Gew.-% Chrom durch Aktivieren mit Natriumhydroxid-Lösung und anschließendem Waschen mit destilliertem Wasser gewonnen.

GB 653,348 offenbart ein Verfahren zur Herstellung von Hexamethylendiamin durch Hydrierung von 1,4-Dicyanobuten an einem Raney-Cobalt Katalysator. In den Beispielen wird die Hydrierung von 1,4-Dicyano-2-buten in verschiedenen Lösungsmitteln (Methanol, Dioxan) in Abwesenheit von Ammoniak oder einer basischen Alkali- oder Erdalkaliverbindung durchgeführt.

Derwent Abstract XP002188943 von JP 63/170342 offenbart die Hydrierung von Nitrilen zu Aminen an Raney-Cobalt-Katalysatoren bei Raumtemperatur bis zu 200 °C und bis zu 100 bar Wasserstoff.

FR-A 2 248 265 offenbart die Hydrierung von aliphatischen, cycloaliphatischen oder aromatischen Dinitrilen zu den entsprechenden Diaminen an Raney-Cobalt-Katalysatoren in Gegenwart von Aminen bei 50 bis 300 bar Wasserstoff und 50 bis 200 °C, gegebenenfalls in einem Lösungsmittel wie Tetrahydrofuran oder Dioxan.

WO 00/27525 offenbart Raney-Katalysatoren, die Eisen und Cobalt und ein drittes Metall wie Molybdän, Chrom, Zirkon und Titan enthalten. Die Katalysatoren werden zur Hydrierung von Adiponitril eingesetzt, wobei in Gegenwart von wässrigem Ammoniak gearbeitet wird.

Aufgabe der Erfindung ist es, ein einfach durchzuführendes Verfahren zur schonenden Hydrierung von Nitrilen bereitszustellen. Aufgabe der Erfindung ist es insbesondere, ein solches Verfahren bereitzustellen, das eine hohe Selektivität bezüglich der Bildung primärer Amine aufweist und bei dem Nebenreaktionen wie die Spaltung der Nitrile vermieden werden.

Gelöst wird die Aufgabe durch ein Verfahren zur kontinuierlichen Hydrierung von Nitrilen zu primären Aminen in flüssiger Phase an einem suspendierten, aktivierten Raney-Katalysator auf Basis einer Legierung aus Aluminium und mindestens einem Übergangsmetall, ausgewählt aus der Gruppe bestehend aus Eisen, Cobalt und Nickel, und gegebenenfalls einem oder mehreren weiteren Übergangsmetallen, ausgewählt aus der Gruppe bestehend aus Titan, Zirkonium, Chrom und Mangan, wobei in Abwesenheit von Ammoniak und basischen Alkali- oder Erdalkaliverbindungen gearbeitet wird. Das Verfahren ist dadurch gekennzeichnet, dass die Katalysatoren (a) durch Behandeln mit wässriger Alkalimetallhydroxid-Lösung, (b) Spülen des Katalysators mit wässriger Alkalimetallhydroxid-Lösung und (c) Spülen des Katalysators mit Wasser aktiviert werden.

In dem erfindungsgemäßen Verfahren bevorzugt eingesetzte Katalysatoren sind auf Basis einer Legierung aus Aluminium und einer festen Lösung aus Nickel in Cobalt oder Cobalt in Nickel, die 0,05 - 50 Gew.-% des gelösten Metalls und gegebenenfalls 0,05 bis 5 Gew.-% des oder der weiteren Übergangsmetalle enthält, hergestellt.

Bevorzugte Übergangsmetalle sind Nickel und Cobalt, besonders bevorzugt sind feste Lösungen von Nickel in Cobalt oder Cobalt in Nickel, die das gelöste Metall in einer Konzentration von 0,05 bis 50 Gew.-% enthalten. Zur Erhöhung der Aktivität und Selektivität enthält die Legierung noch mindestens ein weiteres Übergangsmetall, ausgewählt, aus Titan, Zirkonium, Chrom und Mangan als Promotor, im allgemeinen in Konzentrationen von 0,01 bis 15 Gew.-%, vorzugsweise 0,05 bis 5 Gew.-%, bezogen auf die Gesamtmenge der Übergangsmetalle. Das Gewichtsverhältnis von Aluminium zu Übergangsmetall liegt in der Regel im Bereich von 30 bis 70 Gew.-% Aluminium und 30 bis 70 Gew.-% Übergangsmetall.

Die Herstellung der Aluminiumlegierung erfolgt in an sich bekannter Weise, beispielsweise wie in DE 21 59 736 beschrieben, deren Inhalt bezüglich der Herstellung von Legierungen aus Aluminium und den genannten Übergangsmetallen durch Bezugnahme in die vorliegende Anmeldung einbezogen wird.

Die , Aktivierung der calcinierten Formkörper wird erfindungsgemäß mit einem Alkalimetallhydroxid, vorzugsweise Lithiumhydroxid, Natriumhydroxid, Kaliumhydroxid oder Cäsiumhydroxid oder deren Gemische, besonders bevorzugt mit Natriumhydroxid allein oder im Gemisch mit den oben genannten Alkalimetallhydroxiden, durchgeführt. Hierzu werden wässrige Lösungen des Alkalimetallhydroxids, bevorzugt Natronlauge, verwendet, wobei die Alkalilauge im allgemeinen 5 bis 30 gew.-%ig, bevorzugt 15 bis 25 gew.-%ig ist. Das Molverhältnis von Alkalimetallhydroxid zu Aluminium beträgt in der Regel ungefähr 1 : 1 bis ungefähr 5 : 1, vorzugsweise ungefähr 1,5 : 1 bis ungefähr 3 : 1.

Die Temperatur der Aktivierung beträgt üblicherweise ungefähr 25°C bis ungefähr 106°C, vorzugsweise ungefähr 45°C bis ungefähr 90°C.

Die Dauer der Aktivierung hängt im wesentlichen vom gewünschten Endgehalt an Aluminium ab und liegt im Bereich von 1 bis 10 Stunden, vorzugsweise 2 bis 5 Stunden. Die Aktivierungsprozedur kann auch mehrfach durchgeführt werden.

Nach der Aktivierungsprozedur wird der Katalysator-Formkörper zuerst mit wässriger Allcalimetallhydroxid-Lösung und anschließend mit Wasser gespült.

Bevorzugt wird mit einer wässrigen Lösung des Alkalimetallhydroxids gespült, die 5 bis 30 gew.-%ig, bevorzugt 15 bis 25 gew.-%ig ist. Besonders bevorzugt wird zum Spülen das gleiche Alkalimetallhydroxid wie bei der Aktivierung des Katalysators eingesetzt, und ist die Alkalimetallhydroxid-Konzentration der Spüllösung die gleiche wie in der zur Aktivierung eingesetzten Alkalimetallhydroxid-Lösung. Das Spülen wird vorzugsweise bei Raumtemperatur durchgeführt. Dabei kann das Spülen mit Alkalimetallhydroxid-Lösung mehrfach, vorzugsweise 3-fach durchgeführt werden, wobei die Gesamtmenge der eingesetzten Alkalihydroxid-Lösung ähnlich groß wie bei der Aktivierung ist. Erst danach erfolgt das Spülen mit Wasser, vorzugsweise solange, bis ein pH-Wert des Spülwassers Wert von ca. 8 erreicht ist.

Die gewaschenen, aktivierten Katalysator-Formkörper werden unter Wasser, vorzugsweise in einer Mischung aus Wasser und Methanol aufbewahrt.

Erfindungsgemäß können beliebige Nitrile zu den entsprechenden pirmären Aminen hydriert werden. Bevorzugte Nitrile, die nach dem erfindungsgmäßen Verfahren hydriert werden, sind sogenannte Streckemitrile und Nitrile, die durch Michael-Addition erhalten werden können, der allgemeinen Formeln (I) bzw. (II) 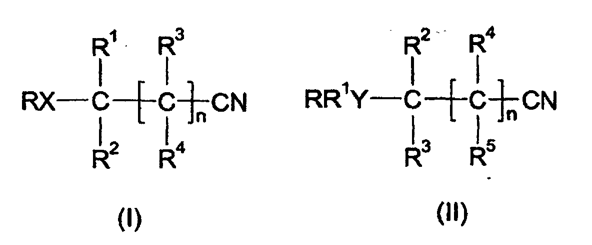 worin
- X: O oder S,
- Y: N oder P,
- n: 0 oder 1 und
- R, R¹, R², R³, R⁴, R⁵: unabhängig voneinander H oder einen gegebenenfalls substituierten Alkylrest mit 1 - 10 C-Atomen oder einen gegebenenfalls substituierten Arylrest mit 6 - 12 C-Atomen
bedeuten.

Beispiele sind Iminodiacetonitril, Nitrilotrisacetonitril, Ethylendiamintetracetonitril, Biscyanoethylpiperazin, Dimethylaminopropionitril, Dimethylaminopropylaminopropionitril und Methoxypropionitril.

Bevorzugte Nitrile sind ferner cyclische Iminonitrile und acyclische Iminonitrile der allgemeinen Formeln (III) und (IV) 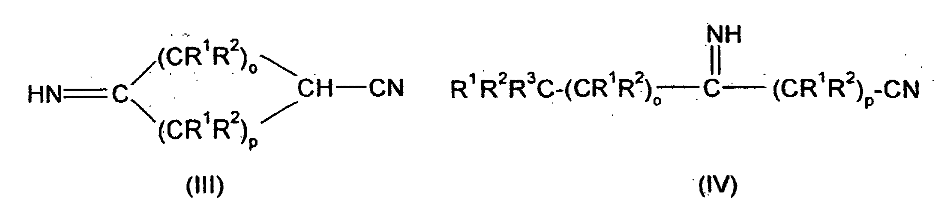 worin
- R¹, R² und R³: unabhängig voneinander H oder einen gegebenenfalls substituierten Alkylrest mit 1 - 10 C-Atomen oder einen gegebenenfalls substituierten Arylrest mit 6 - 12 C-Atomen und
- o, p: 0, 1, 2, 3 ,4 oder 5
bedeuten.

### Ein Beispiel ist Isophoronnitrilimin.

Weitere wichtige Nitrile und Aminonitrile, die nach dem erfindungsgemäßen Verfahren hydriert werden können, sind die nachstehenden Verbindungen (V) bis (XXXII):

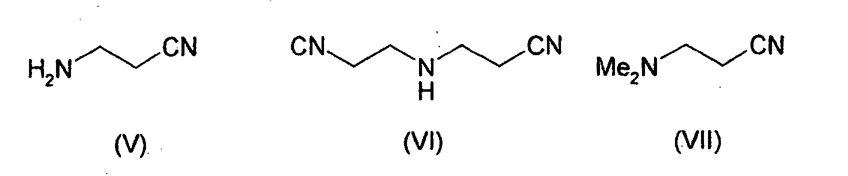

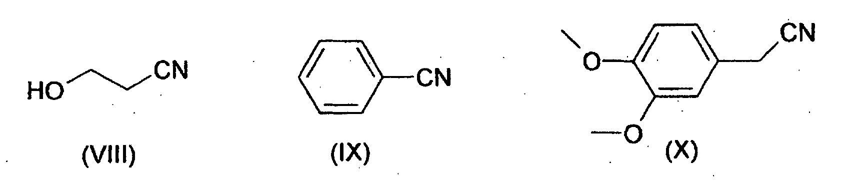

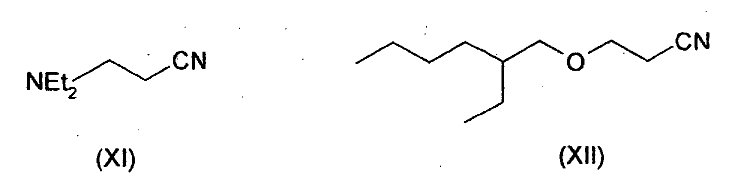

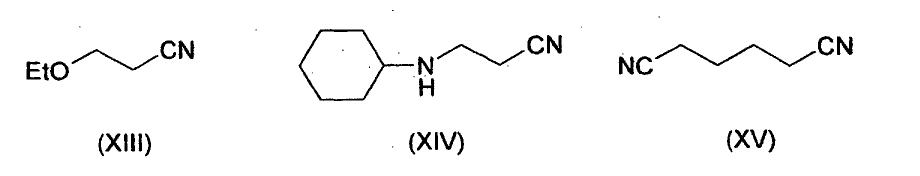

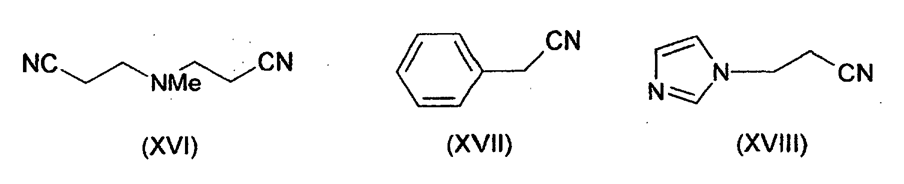

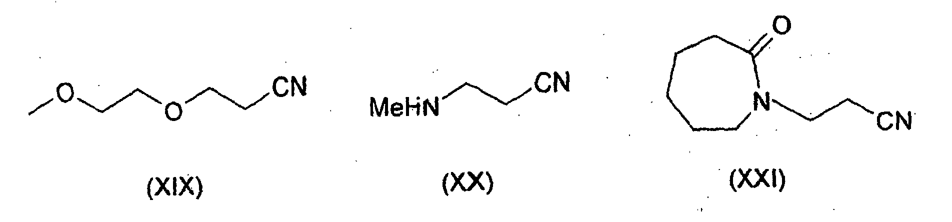

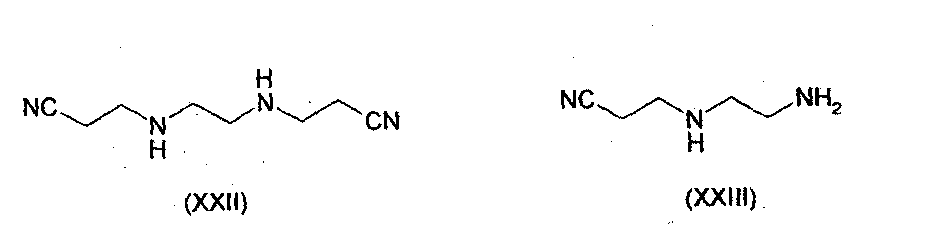

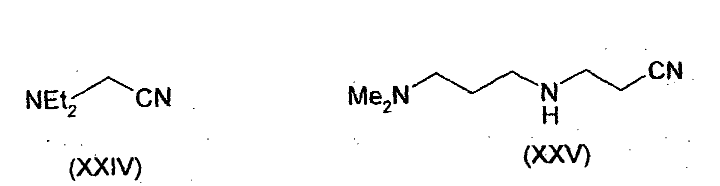

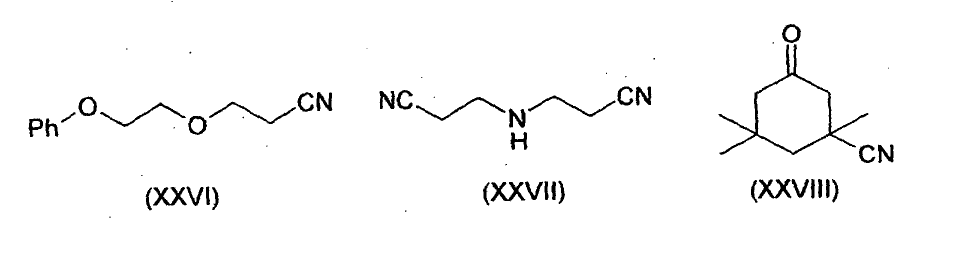

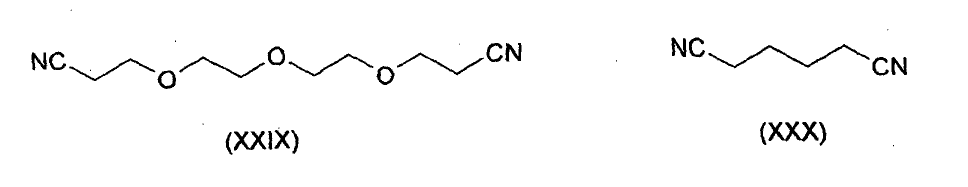

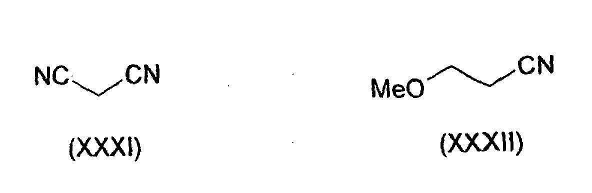

Die Hydrierung wird kontinuierlich mit suspendiertem, in auf- und abwirbelnder Bewegung befindlichem Katalysator durchgeführt. Dinitrile oder Polynitrile können auch teilweise hydriert werden. Durch Veränderung der Verweilzeit können der Umsatz und damit die Produktverhältnisse von Aminen zu Aminonitrilen gezielt eingestellt werden.

Die Hydrierung wird mit suspendiertem Katalysator durchgeführt. Geeignete Reaktoren für die Hydrierung in Suspensions-Fahrweise sind Rührkessel, Strahlschlaufenreaktoren oder Blasensäulen. Die Temperatur beträgt im allgemeinen von 25 bis 125°C, der Wasserstoffdruck im allgemeinen von 10 bis 300 bar. Die zu hydrierenden Nitrile können in einem organischen Lösungsmittel gelöst vorliegen. Bevorzugte organische Lösungsmittel sind aliphatische Alkohole, Amine, Amide wie N-Methylpyrollidon und Dimethylformamid, Ether oder Ester. Die Katalysatorbelastung liegt im allgemeinen zwischen 0,5 und 20, vorzugsweise zwischen 2 und 10 Mol Nitril/l_{Katalysator}*h.

Die Erfindung wird durch das nachstehende Beispiele näher erläutert.

### Beispiel

In eine kontinuierlich betriebene Laborapparatur (150 ml-Autoklav mit Fritte und Doppelflügelrührer) werden 10 g mit Nickel und Chrom dotiertes Raney-Cobalt (Raney® 2724 der Fa. Grace Davison, Chattanooga, Tennessee, USA) gegeben. Der Katalysator enthält 97,1 Gew.-% Co, 3,3 Gew.-% Al, 2,6 Gew.-% Ni, 2,1 Gew.-% Cr und 0,3 Gew.-% Fe. Anschließend wird der Reaktor mit Dimethylformamid geflutet, auf 100°C aufgeheizt, und Iminodiacetonitril als 20 gew.-%ige Lösung in Dimethylformamid mit 20 Nl/h Wasserstoff bei 190 bar bei einer Katalysator-Belastung von 0, 15kg/l(Katalysator)*h hydriert. Bei einem Umsatz von 100% wurde Diethylentriamin mit einer Selektivität von 82% gebildet.

## Patentansprüche

1. Verfahren zur kontinuierlichen Hydrierung von Nitrilen zu primären Aminen in flüssiger Phase an einem suspendierten, aktivierten Raney-Katalysator auf Basis einer Legierung aus Aluminium und mindestens einem Übergangsmetall, ausgewählt aus der Gruppe bestehend aus Eisen, Cobalt und Nickel, wobei in Abwesenheit von Ammoniak und basischen Alkali- oder Erdalkaliverbindungen gearbeitet wird, **dadurch gekennzeichnet, dass** der Katalysator aktiviert wird durch (a) Behandeln mit wässriger Alkalimetallhydroxid-Lösung, (b) Spülen des Katalysators mit wässriger Alkalimetallhydroxid-Lösung und (c) Spülen des Katalysators mit Wasser.

2. Verfahren nach Anspruch 1, **dadurch gekennzeichnet, dass** die Legierung ein oder mehrere weitere Übergangsmetalle enthält, ausgewählt aus der Gruppe bestehend aus Titan, Zirkonium, Chrom und Mangan.

3. Verfahren nach Anspruch 1 oder 2, **dadurch gekennzeichnet, dass** der Katalysator auf Basis einer Legierung aus Aluminium und einer festen Lösung aus Nickel in Cobalt oder Cobalt in Nickel, die 0,05 - 50 Gew.-% des gelösten Metalls und gegebenenfalls 0,05 bis 5 Gew.-% des oder der weiteren Übergangsmetalle enthält, hergestellt ist.

4. Verfahren nach einem der Ansprüche 1 bis 3, **dadurch gekennzeichnet, daß** das zu hydrierende Nitril in einem organischen Lösungsmittel gelöst vorliegt.

5. Verfahren nach Anspruch 4, **dadurch gekennzeichnet, daß** das organische Lösungsmittel Dimethylformamid oder N-Methylpyrrolidon ist.

6. Verfahren nach einem der Ansprüche 1 bis 5, **dadurch gekennzeichnet, daß** die Hydrierung bei einer Temperatur von 25 bis 125 °C und einem Wasserstoffdruck von 10 bis 300 bar durchgeführt wird.

7. Verfahren nach einem der Ansprüche 1 bis 6, **dadurch gekennzeichnet, daß** die Hydrierung in einem Rührkessel, Strahlschlaufenreaktor oder einer Blasensäule durchgeführt wird.

8. Verfahren nach einem der Ansprüche 1 bis 7, **dadurch gekennzeichnet, daß** das Nitril ausgewählt ist aus Strecker-Nitrilen oder Michael-Addukten an Acrylniril.

9. Verfahren nach einem der Ansprüche 1 bis 7, **dadurch gekennzeichnet, daß** das Nitril ausgewählt ist aus der Gruppe bestehend aus Iminodiacetonitril, Nitrilotrisacetonitril, Ethylendiamintetraacetonitril, Biscyanoethylpiperazin, Dimethylaminopropionitril, Dimethylaminopropylaminopropionitril, Isophoronnitrilimin und Methoxypropionitril.

## Claims

1. A process for the continuous hydrogenation of nitriles to primary amines in the liquid phase over a suspended, activated Raney catalyst based on an alloy of aluminum and at least one transition metal selected from the group consisting of iron, cobalt and nickel, the hydrogenation being carried out in the absence of ammonia and basic alkali metal compounds or alkaline earth metal compounds wherein the catalyst is activated by (a) treatment with aqueous alkali metal hydroxide solution, (b) rinsing the catalyst with aqueous alkali metal hydroxide solution and (c) rinsing the catalyst with water.

2. A process as claimed in claim 1, wherein the alloy further comprises one or more additional transition metals selected from the group consisting of titanium, zirconium, chromium and manganese.

3. A process as claimed in claim 1 or 2, wherein the catalyst has been produced on the basis of an alloy of aluminum and a solid solution of nickel in cobalt or of cobalt in nickel which comprises 0.05-50% by weight of the dissolved metal and, if desired, from 0.05 to 5% by weight of one or more of the further transition metals.

4. A process as claimed in any of claims 1 to 3, wherein the nitrile to be hydrogenated is present as a solution in an organic solvent.

5. A process as claimed in claim 4, wherein the organic solvent is dimethylformamide or N-methylpyrrolidone.

6. A process as claimed in any of claims 1 to 5, wherein the hydrogenation is carried out at from 25 to 125°C and a hydrogen pressure of from 10 to 300 bar.

7. A process as claimed in any of claims 1 to 6, wherein the hydrogenation is carried out in a stirred vessel, a jet loop reactor or a bubble column.

8. A process as claimed in any of claims 1 to 7, wherein the nitrile is selected from the group consisting of extender nitriles and Michael adducts of acrylonitrile.

9. A process as claimed in any of claims 1 to 7, wherein the nitrile is selected from the group consisting of iminodiacetonitrile, nitrilotriacetonitrile, ethylenediaminetetraacetonitrile, biscyanoethylpiperazine, dimethylaminopropionitrile, dimethylaminopropylaminopropionitrile, isophoronenitrilimine and methoxypropionitrile.

## Revendications

1. Procédé d'hydrogénation en continu de nitriles en amines primaires en phase liquide sur un catalyseur de Raney en suspension activé à base d'un alliage d'aluminium et d'au moins un métal de transition, choisi dans le groupe formé par le fer, le cobalt et le nickel, dans lequel on travaille en l'absence d'ammoniac et de composés alcalins ou alcalino-terreux basiques, **caractérisé en ce que** le catalyseur est activé par (a) un traitement avec une solution aqueuse d'hydroxyde de métal alcalin, (b) un rinçage du catalyseur avec la solution aqueuse d'hydroxyde de métal alcalin et (c) un rinçage du catalyseur avec de l'eau.

2. Procédé selon la revendication 1, **caractérisé en ce que** l'alliage contient un ou plusieurs autres métaux de transition, choisis dans le groupe formé par le titane, le zirconium, le chrome et le manganèse.

3. Procédé selon la revendication 1 ou 2, **caractérisé en ce que** le catalyseur est produit à partir d'un alliage d'aluminium et d'une solution solide de nickel dans du cobalt ou de cobalt dans du nickel, qui contient 0,05% à 50% en poids du métal dissous et éventuellement 0,05% à 5% en poids du ou des autres métaux de transition.

4. Procédé selon l'une quelconque des revendications 1 à 3, **caractérisé en ce que** le nitrile à hydrogéner se présente sous une forme dissoute dans un solvant organique.

5. Procédé selon la revendication 4, **caractérisé en ce que** le solvant organique est le diméthylformamide ou la N-méthylpyrrolidone.

6. Procédé selon l'une quelconque des revendications 1 à 5, **caractérisé en ce que** l'on réalise l'hydrogénation à
une température allant de 25°C à 125°C et sous une pression d'hydrogène allant de 10 bars à 300 bars.

7. Procédé selon l'une quelconque des revendications 1 à 6, **caractérisé en ce que** l'on réalise l'hydrogénation dans un récipient sous agitation, dans une colonne à écoulement en boucle à faisceau ou dans une colonnes à bulles.

8. Procédé selon l'une quelconque des revendications 1 à 7, **caractérisé en ce que** le nitrile est choisi parmi les nitriles issus de la réaction de Strecker ou les produits d'addition de la réaction de Michaël à l'acrylonitrile.

9. Procédé selon l'une quelconque des revendications 1 à 7, **caractérisé en ce que** le nitrile est choisi dans le groupe formé par l'iminodiacétonitrile, le nitrilotrisacétonitrile, l'éthylènediaminetétraacétonitrile, la biscyanoéthylpipérazine, le diméthylaminopropionitrile, le diméthylaminopropylaminopropionitrile, l'isophoronenitrilimine et le méthoxypropionitrile.
